# EUROPEAN PATENT APPLICATION

(11) **EP 2 062 568 A1**
(43) Date of publication of application: **27.05.2009**
(21) Application number: 07397042.8
(22) Date of filing: 22.11.2007
(51) Int. Cl.: A61K 9/00

(54) **Vaginal delivery system**

(71) Applicant: Bayer Schering Pharma Oy, 20210 Turku (FI)
(72) Inventor: Talling, Christine, 20610 Turku (FI); Hanes, Vladimir, Tarrytown, NY 10591 (US); Keinänen, Antti, 20540 Turku (FI); Holmberg, Svante, 20900 Turku (FI); Nikander, Hannu, 21330 Paattinen (FI)
(74) Representative: Matilainen, Mirja Helena

(57) **Abstract**

The present invention is related to an intravaginal delivery system for the controlled release of a progestogen and an estrogen, comprising additionally a therapeutically active or a health-promoting substance (1) capable of giving and/or enhancing the protection against bacterial and fungal infections, and/or enhancing the protection against sexually transmitted diseases. The delivery system consists of one or more compartments (2,4,5), one of each comprising a core (7) and a membrane (3) encasing the core, said core and membrane essentially consisting of a same or different polymer composition, wherein at cast one of the cores comprises a progestogen or a mixture of a progestogen and an estrogen, and another core may comprise an estrogen or a progestogen, and wherein the membrane or the surface of the membrane or at least one of the cores comprises said therapeutically active or a health-promoting substance.

## Description

The present invention is related to an improved intravaginal delivery system for the controlled release of a progestogen and an estrogen, additionally comprising a therapeutically active or a health-promoting substance capable of giving and/or enhancing the protection against bacterial and fungal infections, and/or enhancing the protection against sexually transmitted diseases. The delivery system consists of one or more compartments each comprising a core and a membrane encasing the core, said core and membrane essentially consisting of a same or different polymer composition, wherein at least one of the cores comprises a progestogen or a mixture of a progestogen and an estrogen, and another core may comprise an estrogen or a progestogen, and wherein the membrane or the surface of the membrane or at least one of the cores comprises said therapeutically active or a health-promoting substance.

### Background

Vaginal delivery systems capable of releasing two or more therapeutically active substances at a substantially constant rate to one another over a prolonged period of time are extremely useful for certain applications, for example contraception and hormone replacement therapy. Extensive use has been made of the simultaneous administration of an agent having a progestogenic activity and an agent having an estrogenic activity, preferably in a substantially constant ratio. Contraceptive reliability is mainly provided by the progestogenic component and the estrogen component acts to increase the ovulation inhibitory effect of progestin and to ensure cycle stability.

A number of different constructions of vaginal rings are known from the literature, see for example US 4,596,576 and in US 4,237,885. In basic solutions the delivery system is simply formed by a drug-containing polymer core in the form of a closed ring. A modification of this is a closed ring which comprises a drug-free core surrounded by a drug matrix optionally containing a number of different drugs and in addition optionally an outermost polymer membrane.

EP 876815 relates to a preferably ring-shaped vaginal delivery system for the simultaneous release of a progestogenic steroid compound and an estrogenic steroid compound in a fixed physiological ratio over a prolonged period of time. The delivery system comprises at least one compartment comprising a thermoplastic polymer core containing the mixture of the progestogenic and estrogenic compounds and a thermoplastic polymer skin, the progestogenic compound being initially dissolved in the polymer core material in a relatively low degree of supersaturation. The drug delivery device is physically stable only when stored below room temperature. As indicated in EP 876815 the progestogen may eventually crystallize out on the exterior surface of the vaginal ring. Such a crystallization of progestogen onto the skin of the device may lead to uncontrolled and high burst release.

EP 836473 relates to a ring-shaped device comprising a first compartment having a non-medicated core of ethylene-vinylacetate copolymer, encircled by a steroid hormone loaded ethylene-vinylacetate copolymer layer, and a non-medicated outer layer of ethylene-vinylacetate copolymer; a second compartment comprising a core of ethylene-vinylacetate copolymer loaded with a steroid hormone and a non-medicated outer layer of ethylene-vinylacetate polymer; and optionally placebo compartments of a thermoplastic material separating the first from the second compartment. In a preferred embodiment the invention is related to a two-compartment vaginal ring with the first compartment comprising crystalline etonogestrel and the second compartment comprising a (sub)-saturated mixture of etonogestrel and ethinyl estradiol, both compartments optionally being separated from each other by placebo compartments of high density polyethylene.

WO 1995000199 is related to an intravaginal delivery system comprising a flexible support means, and a delivery means carried by the support means and containing active agent. The support means consists of a core member substantially in the form of an open ring, and the delivery means consists of at least one sleeve-like polymer body which encircles the core member in a belt wise manner along a part of the length of the core member.

WO 2005089723 relates to a drug delivery system consisting of one or more compartments and comprising a progestogenic compound dissolved in a thermoplastic polyethylene vinylacetate copolymer whereby, if the delivery system consists of one compartment, the compartment comprises (i) a core of a thermoplastic polyethylene vinylacetate copolymer comprising the progestogenic compound, such progestogenic compound being dissolved in the polyethylene vinylacetate copolymer up to a concentration below the saturation level at 25°C, and an estrogenic compound; and (ii) a skin of a thermoplastic polyethylene vinylacetate copolymer covering the core, said skin being permeable for both compounds;-if the delivery system consists of more than one compartment, only one compartment comprises (iii) the progestogenic compound, such progestogenic compound being dissolved in a core of a thermoplastic polyethylene vinylacetate copolymer up to a concentration below the saturation level at 25°C, and an estrogenic compound; and (iv) a skin of a thermoplastic polyethylene vinylacetate copolymer covering the core, said skin being permeable for both compounds.

EP 862396 relates to a vaginal ring containing a body made of a first polymeric material having at least one hollow internal channel defining an opening to the exterior of said body and which channel is adapted to receive a drug-containing core through said opening, and a core containing at least one intravaginally administrable drug dispersed in a second polymeric material disposed in the channel. The core is positioned in the vaginal ring body suitably prior to use in order to substantially avoid initial bursts of drug into the tissues of the subject and resultant side effects such as nausea and vomiting.

The advantage of vaginal rings in general is that the woman is free from the necessity of having to take tablets daily. The ring-shaped structure is simple to apply, it is well tolerated and at any time the device can easily be removed and reinserted by the woman herself. A significant drawback of these devices is that they do not give any protection against bacterial and fungal infections and/or against sexually transmitted diseases.

Therefore there is still need for an improved intravaginal delivery system, which in addition to a sufficient daily dosage of a progestogen and an estrogen needed for contraception or hormone replacement therapy, would release a sufficient amount of a therapeutically active or a health-promoting substance capable of giving and/or enhancing the protection against bacterial and fungal infections, and/or enhancing the protection against sexually transmitted diseases.

The resident microorganisms are known to be the main factors to maintain and stabilize the physiological environment in the vagina. Lactobacilli are the predominant microorganisms in the vaginal bacteria, and they play a major role in maintaining a healthy urogenital tract. They are capable of preventing adhesion and growth of pathogenic microorganisms through mechanisms that appear to involve secretion of anti-adhesion factors, hydrogen peroxide, bacteriocins lethal to pathogens and fermenting the glycogen derived from the decline of the atrophic vaginal mucosa, to lactic acid with release of hydrogen ions, the final result being the optimal pH value (Microb. Infect. 4, 319 324 (2002)).

Vaginal pH undergoes physiologically changes from birth to menopause, according to changes of ovarian steroids occurring during woman's life. Adequate levels of estrogens play a role in the trophism of vaginal mucosa, and estrogens increase the cellular content of glycogen. Several factors, such as sexual activity, oral contraceptives or systemic or local therapies may increase vaginal pH through different mechanisms. The change in the pH value may also be indicative to presence of disbalance in the vaginal environment such as systemic diseases or vaginal infections. The increase of vaginal pH above 4.0-4.5 is detrimental for the survival of Lactobacillus bacteria, but not for other micro organisms, especially for the pathogenetic microorganisms, whose replication on the contrary is favored by the absence of contraction exerted by lactobacilli.

Probiotic agents have been used to adjust or to maintain the normal vaginal pH value. For example, international patent application WO 2006/65873 relates to a fiber-reinforced composite ring containing ferrous gluconate or ferrous ascorbate as an active agent and acids, such as ascorbic and glycolic acids to maintain the vaginal pH below 6 and preferably at 3-4.5.

International patent application WO 2002/15832 relates to a non-hormonal intravaginal device and the vagina is maintained at a pH of about 5-6 by the addition of ascorbic acid to the matrix hydrogel. WO 2006/17341 relates to a vaginal device partly or completely coated or covered by or combined with a mucoadhesive composition containing a therapeutical agent and a health-promoting agent such as ascorbic acid.

The delivery of biological substances such as probiotics and bacteria in general are described for example in WO 2003/26687, US 20050152966 and US 20030096002, based on a swellable polymer matrices, in WO 2002/94224 based on biocompatible carbohydrate polymers associated with milk protein, and in WO 2005/74976 based on uncrosslinked starch.

### OBJECT OF THE INVENTION

The object of the present invention is to provide an improved intravaginal delivery system for the controlled release of progestogen and estrogen, and additionally a therapeutically active or a health-promoting substance capable of giving and/or enhancing the protection against bacterial and fungal infections, and/or enhancing the protection against sexually transmitted diseases. The delivery system consists of one or more compartments each comprising a core and a membrane encasing the core, said core and membrane essentially consisting of a same or different polymer composition, wherein at least one of the cores comprises a progestogen or a mixture of a progestogen and an estrogen, or an estrogen, and wherein the surface of the membrane, the membrane or at least one of the cores comprises said therapeutically active or health-promoting substance.

The present invention especially provides an intravaginal delivery system for the simultaneous administration of a sufficiently high daily dosage of drospirenone (6β,7β;15β;16β-dimethylene-3-oxo-17α-preg-4-ene-21,17-carbolactone), an estrogen, preferably estradiol, estradiol hemihydrate , an estradiol ester or ethinyl estradiol and a therapeutically active or a health-promoting substance capable of giving and/or enhancing the protection against bacterial and fungal infections and/or enhancing the protection against sexually transmitted diseases, preferably a representative of Lactobacillus species.

The drug delivery system according to the invention is especially suitable for use in the field of female contraception and hormone replacement therapy. The delivery system can also be used for treating diseases, disorders and symptoms associated for example with natural menopause, peri-menopause, post-menopause, hypogonadism or primary ovarian failure in women, wherein the amount of estrogen is sufficient to treat diseases, disorders and symptoms associated with deficient endogenous levels of estrogen and the amount of drospirenone is sufficient to protect the endometrium from the adverse effects of estrogen. The delivery system can further be used for the treatment of endometriosis and uterine fibroids based on the suppression of endogenous sexual steroid production combined with exogenous progestogene effects. Further, the invention provides a convenient and highly adaptable drug delivery system for use in female animals, too.

In addition to contraception or hormone replacement therapy, the invention provides a method for giving and/or enhancing the protection against bacterial and fungal infections and/or enhancing the protection against sexually transmitted diseases, which comprises the steps of positioning the delivery system of the subject invention within the female vaginal tract and retaining the system a prolonged period of time within the vaginal tract. Thus the present invention concerns a delivery system and a method as described below in the independent claims.

### BRIEF DESCRIPTION OF THE FIGURES

The invention is further illustrated by the following examples, describing various constructions of the intravaginal delivery system according to the invention

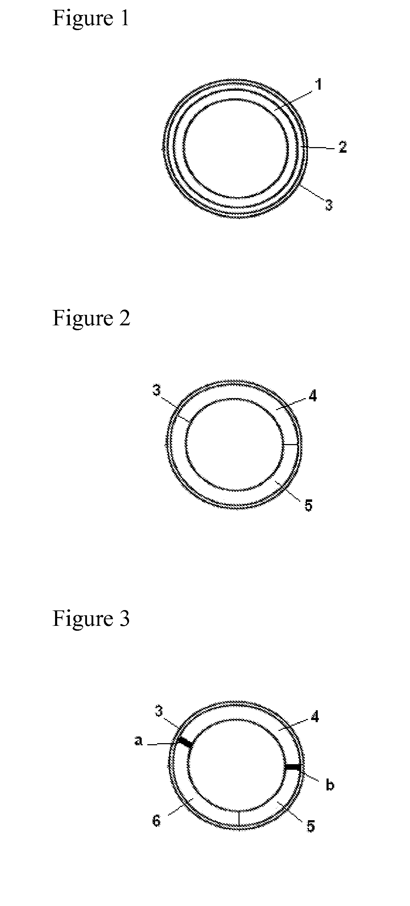

Figure 1 is an intravaginal delivery system comprising a supporting ring free of active agent or the first compartment containing a therapeutically active agent (1), the second compartment (2) applied to the outer surface of 1 and containing a therapeutically active agent and a membrane layer (3) encasing the whole delivery system or a part of it. The supporting ring or compartment 1 may have a groove at least on the portion of the annular surface adapted to mate with corresponding compartment 2.

Figure 2 is an intravaginal delivery system comprising two compartments 4 and 5 encased by a membrane 3, the compartments being positioned next to each other

Figure 3 is an intravaginal delivery system comprising three compartments 4, 5 and 6 encased by a membrane 3, the compartment 4 is separated from other compartments by separation membranes a and b, while compartments 5 and 6 are positioned next to each other

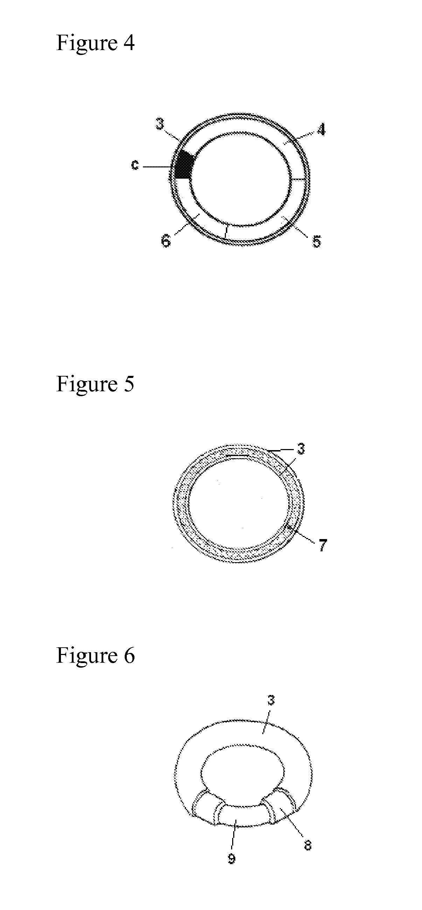

Figure 4 is an intravaginal delivery system comprising three compartments 4, 5 and 6 encased by a membrane 3. An inert placebo compartment c separates the compartments 4 and 6, the compartments 4 and 5 as well as 5 and 6 are positioned next to each other. In this design compartments 4, 5 or 6 may or may not contain a therapeutically active substance, either the same or different.

Figure 5 is a general design of an intravaginal delivery system comprising a core 7 and a membrane 3 encasing the core.

Figure 6 is another type of general design of an intravaginal delivery system comprising a core 8, a membrane 3 encasing the core and an inert supporting member 9

### DETAILED DESCRIPTION

The advantages of the invention are obtained by the intravaginal delivery system consisting of one or more compartments, one or each compartment comprising a core and a membrane encasing the core, said core and membrane essentially consisting of a same or different polymer composition, wherein at least one of said compartments comprises a progestogen or a mixture of a progestogen and an estrogen, and another core may optionally comprise an estrogen or a progestogen, and wherein at least one of the cores or the membrane or the surface of the membrane additionally comprises a therapeutically active or a health-promoting substance capable of giving and/or enhancing the protection against bacterial and fungal infections and/or enhancing the protection against sexually transmitted diseases.

According to an embodiment of the invention, the intravaginal delivery system consists of one compartment comprising a core and a membrane encasing said core, said core and membrane essentially consisting of a same or different polymer composition, wherein the core comprises a mixture of progestogen and an estrogen and the membrane comprises a therapeutically active or a health-promoting substance capable of giving and/or enhancing the protection against bacterial and fungal infections and/or enhancing the protection against sexually transmitted diseases.

According to another embodiment of the invention, the intravaginal delivery system consists of at least two compartments comprising a core and a membrane encasing said core, said core and membrane essentially consisting of a same or different polymer composition, wherein at least one of the cores comprises a mixture of progestogen and an estrogen and one of the cores comprises said therapeutically active or a health-promoting substance.

According to a further embodiment of the invention, the intravaginal delivery system consists of at least two compartments each comprising a core and a membrane encasing said core, said core and membrane essentially consisting of a same or different polymer composition, wherein one of the cores comprises a progestogen or a mixture of a progestogen and an estrogen, another core comprises a progestogen or an estrogen, and the membrane or the surface of the membrane or at least one of the cores comprises a therapeutically active or a health-promoting substance capable of giving and/or enhancing the protection against bacterial and fungal infections and/or enhancing the protection against sexually transmitted diseases.

Any suitable design of the delivery system or any combination of structure is naturally possible and within the scope of the invention.

The core consists essentially of an polymer composition, that is, the core is an elastomer matrix wherein the therapeutically active substance or substances are dispersed. Therefore, even if the membrane encasing the core would be damaged, the therapeutically active substances would not be released in a completely uncontrolled manner causing side effects to the patient. Thus the polymer composition of the core is preferably chosen so that the membrane primarily regulates the release of the therapeutically active agent. The release rates in general can be controlled by the membrane alone or by the membrane together with the core. It is also possible that the release rate is mainly controlled by the core.

According to the embodiment in which the delivery system consists of two or more compartments, said compartments may be positioned next to each other. The compartments may also be side-by-side or one on the other, for example as described in US 4,822,616 and US 4,012,496 by Schering AG, the upper compartment being assembled on or encircling the surface of the lower compartment or assembled in a groove on the surface of the lower compartment. The length of the compartments may be same or different. The compartments may or may not be separated from each other by a separation membrane or by an inert placebo compartment. An advantage of using several compartments separated from each other by a membrane or an inert placebo compartment is that the release rates are more easily controllable since there is no interaction between the active substances.

The membrane may cover the whole delivery system or cover only a part of the system, whereby the degree of extension can vary depending on a number of factors, for example such as the choice of materials and the choice of active agents. The thickness of the membrane depends on materials and active agents used as well as on desired release profiles, but generally the thickness is smaller the thickness of the core member. The membrane may consist of more than one layer, in which case one of the layers or several layers may comprise a therapeutically active or a health-promoting substance capable of giving and/or enhancing the protection against bacterial and fungal infections and/or enhancing the protection against sexually transmitted diseases. Each layer has a certain thickness, and the thickness of the layers may be the same or different.

The outer surface or membrane may further have different designs, layers or holes , in which case one of the layers or holes may comprise a therapeutically active or a health-promoting substance capable of giving and/or enhancing the protection against bacterial and fungal infections and/or enhancing the protection against sexually transmitted diseases. Each layer has a certain thickness, either the same or different, and each hole may have certain depth. The combination of different membrane layers either in design, thickness or in material or both, gives a further possibility for controlling the release rates of the active agents. The surface of at least one of the membranes may also comprise a therapeutically active or a health-promoting substance in a form of particles, crystals, microcrystals, powder, suspension or a like. The polymer composition used in the membrane is such that it allows the pre-determined release rates of the therapeutically active agents.

Polymer compositions of the core, the membrane and the possible separation membrane or the inert placebo compartment, can be the same or different and may stand for one single polymer, a mixture of polymers or the polymer composition may be made up of polymers that are blended with each other.

In principle any polymer, either biodegradable or non-biodegradable, can be used as long as it is biocompatible. As known in the art, the release kinetics of a therapeutically active agent from a polymer based delivery system depends on the molecular weight, solubility, diffusivity and charge of the therapeutically active agent as well as on the characteristics of the polymer, on the percentage of the loading of the therapeutically active agent, on the distance the therapeutically active agent must diffuse through the device body to reach its surface and on the characteristics of any matrix or membrane.

Polysiloxanes, in particular poly (dimethyl siloxane) (PDMS), are highly suitable for use as a membrane or matrix regulating the permeation rate of drugs. Polysiloxanes are physiologically inert, and a wide group of drugs are capable of penetrating polysiloxane membranes, which also have the required strength properties. The permeation rate of the drugs can be adjusted at a desired level by modifying the polymeric material in a suitable way, e.g. by adjusting hydrophilic or hydrophobic properties of the material. It is for example known from the literature that addition of poly (ethylene oxide) groups or trifluoropropyl groups to a PDMS polymer may change the permeation rate of the drugs.

Further examples of suitable materials include, but are not limited to, copolymers of dimethylsiloxanes and methylvinylsiloxanes, ethylene/vinyl acetate copolymers (EVA), polyethylene, polypropylene, ethylene/propylene copolymers, acrylic acid polymers, ethylene/ethyl acrylate copolymers, polytetrafluoroethylene (PTFE), polyurethanes, polybutadiene, polyisoprene, poly(methacrylate), polymethyl methacrylate, styrenebutadiene-styrene block copolymers, poly(hydroxyethylmethacrylate) (pHEMA), polyvinyl chloride, polyvinyl acetate, polyethers, polyacrylonitriles, polyethylene glycols, polymethylpentene, polybutadiene, polyhydroxy alkanoates, poly(lactic acid), poly(glycolic acid), polyanhydrides, polyorthoesters, hydrophilic polymers such as the hydrophilic hydrogels , cross-linked polyvinyl alcohol, neoprene rubber, butyl rubber, hydroxyl-terminated organopolysiloxanes of the room temperature vulcanizing type which harden to elastomers at room temperature following the addition of cross-linking agents in the presence of curing catalysts, one- or two-component dimethylpolysiloxane compositions cured by hydrosilylation at room temperature or under elevated temperatures, as well as mixtures thereof.

The structural integrity of the material may be enhanced by the addition of a particulate material such as silica or diatomaceous earth. The elastomers can also be mixed with other additives to adjust elastomer's hydrophilic or hydrophobic properties while taking into account that all additives need to be biocompatible and harmless to the patient. The core or membrane may also comprise additional material to further adjust the release rate of one or several of the therapeutic substances, for example complex forming agents such as cyclodextrin derivatives to adjust the initial burst of the substance to the accepted or desired level. Auxiliary substances, for example such as tensides, antifoaming agents, solubilisers or absorption retarders, or a mixture of any two or more of such substances, can also be added in order to impart the desired physical properties to the body of the delivery system.

According to an embodiment, the core and the membrane are made of a siloxane based elastomer composition comprising at least one elastomer and possibly a non-crosslinked polymer.

The term "elastomer composition" may stand for one single elastomer, the deformation of which caused by the strain is reversible so that the elastomer's shape recovers to a certain level after the strain. The elastomer composition may also be made up of two or more elastomers blended with each other.

The term "siloxane-based elastomer" shall be understood to cover elastomers made of poly (disubstituted siloxanes) where the substituents mainly are lower alkyl, preferably alkyl groups of 1 to 6 carbon atoms, or phenyl groups, wherein said alkyl or phenyl can be substituted or unsubstituted. A widely used and preferred polymer of this kind is poly (dimethylsiloxane) (PDMS).

The elastomer composition may also be selected from the group consisting of
- an elastomer composition comprising poly(dimethylsiloxane) (PDMS),
- an elastomer composition comprising a siloxane-based elastomer comprising 3,3,3-trifluoropropyl groups attached to the silicon atoms of the siloxane units,
- an elastomer composition comprising poly(alkylene oxide) groups, said poly(alkylene oxide) groups being present as alkoxy-terminated grafts or blocks linked to the polysiloxane units by silicon-carbon bonds or as a mixture of these forms, and
- a combination of at least two thereof.

According to a preferred embodiment of the invention, in the siloxane-based elastomer from 1 to approximately 50 % of the substituents attached to the silicon atoms of the siloxane units are 3,3,3-trifluoropropyl groups. The percentage of the substituents that are 3,3,3-trifluoropropyl groups can be for example 5-40 %, 10-35 %, 1-29 % or 15-49.5 %. One polymer of this kind, in which approximately 50 % of the methyl substituents at the silicon atoms are replaced by 3,3,3-trifluoropropyl groups, is commercially available. The term "approximately 50 %" means that the degree of 3,3,3-trifluoropropyl substitution is in fact somewhat below 50 %, because the polymer must contain a certain amount (about 0.15 % of the substituents) of cross-linkable groups such as vinyl or vinyl-terminated groups.

According to an especially preferred embodiment of the invention, the siloxane-based elastomer comprises poly(alkylene oxide) groups so that the poly(alkylene oxide) groups are present in the said elastomer either as alkoxy-terminated grafts of polysiloxane units or as blocks, the said grafts or blocks being linked to the polysiloxane units by silicon-carbon bonds. Preferably the poly(alkylene oxide) groups mentioned above are poly(ethylene oxide) (PEO) groups. In the core or membrane polymer composition the proportion of the polysiloxane comprising poly (alkylene oxide) groups, for example polydimethylsiloxane comprising poly(ethylene oxide) groups as alkoxy-terminated grafts or as blocks that are linked to the polysiloxane units by silicon-carbon bonds (PEO-b-PDMS copolymer) may vary from zero to 80 % of the total amount of polymers, but can naturally be higher.

The methods for the preparation of suitable elastomers are given for example in international patent applications WO 00/00550, WO 00/29464 and WO 99/10412 (each assigned to Leiras Oy).

When the therapeutically active or a health-promoting substance is relatively large molecule, such as for example Lactobacillus species, biodegradable polymers, for example hydrogels, are especially suitable membrane or matrix materials.

Progestogen can be any therapeutically active substance having progestogenic activity enough to achieve contraception or to be useful in hormone replacement therapy. Examples of suitable progestogenic compounds include compounds such as cyproterone acetate, desogestrel, etonogestrel, levonorgestrel, lynestrenol, medroxyprogesterone acetate, norethisterone, norethisterone acetate, norgestimate, gestodene or drospirenone.

In a particular embodiment the progestogenic compound is drospirenone. In place of the active substance itself, an ester or prodrug of drospirenone may be employed in the present composition, e.g. an oxyiminopregnane carbolactone as disclosed in WO 98/24801.

Estrogen may be selected from the group consisting of estradiol, ethinyl estradiol, esters of estradiol such as estradiol valerate, estradiol benzoate and estradiol succinate, estradiol hemihydrate, estradiol sulfamates, estrone, estriol, estriol succinate and conjugated estrogens, including conjugated equine estrogens such as estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequilenin sulfate and 17α-dihydroequilenin sulfate or mixtures thereof. Particularly interesting estrogens are those selected from the group consisting of estradiol, estradiol valerate, estradiol succinate, estradiol benzoate, estradiol hemihydrate, estradiol sulfamates, estrone, and estrone sulfate or mixtures thereof. Most preferred compounds are ethinyl estradiol, estradiol, estradiol hemihydrate, estradiol succinate, estradiol valerate or estradiol benzoate.

Other suitable therapeutically active substances include, but not limited to, compounds which can be used to treat and/or prevent bacterial or fungal infections and/or sexually transmitted diseases, for example antimicrobial agents, antibacterial agents, for example such as metronidazole, clindamycin, ampicillin, amoxicillin, tetracycline, doxycycline, antiviral agents, for example such as acyclovir, famciclovir, ganciclovir, saquinavir, valacyclovir and AZT, various antibiotics, antifungal agents, for example such as conazole derivatives like itraconazole, miconazole, terconazole, isoconazole, fenticonazole, fluconazole, ketoconazole, butoconazole and econazole, clotrimazole, , metronidazole, clindamycin, and 5 fluoracil, anti-inflammatoric agents and the like..

Term "health-promoting agent" means a health-sustaining agent or a health-enhancing agent or generally a substance or a combination of substances that are or may be used for the purpose of maintaining and/or improving health or treating and/or preventing disease conditions. Such compounds broadly include, but are not limited to, vitamins, minerals, enzymes, co-enzymes, co-factors, microorganisms, organic acids, spermicides, detergents, surfactants and a variety of molecules extracted from natural sources such as amino acids, polysaccharides, peptides, naturally occurring hormones and biochemical intermediates, as well as naturally occurring molecules synthesized by chemical or biological means

Preferable substances, but not limited to, are the compounds which can especially be used to maintain and stabilize the physiological environment in the vagina, for example such as natural amino acids, lactic acid, polylactic acids, glycolic acid, polyglycolic acids, carbopol, polycarbophil, ascorbic acid, D-pantothenic acid, folic acid and the reduced forms thereof, especially tetrahydrofolates and metabolites of folic acid, preferably 5-methyl-6(S)-tetrahydrofolic acid and its salts, especially its earth alkaline salts, of these the calcium salt (Metafolin) being preferred, fumaric acid, benzoic acid, p-aminobenzoic acid, alginic acid, sorbic acid, tartaric acid, edetic acid, salts of these acids, niacinamide, Lactobacillus species, for example such as Lactobacillus reuteri, Lactobacillus reuterii RC-14, Lactobacillus delbrueckii, Lactobacillus gasseri, Lactobacillus jensenii, Lactobacillus catenaforme, Lactobacillus paracasei, Lactobacillus paracasei Lbp PB01, Lactobacillus casei, Lactobacillus acidophilus, Lactobacillus acidophilus Lba EB01, Lactobacillus crispatus, Lactobacillus crispatus CTV05, Lactobacillus salivarius, Lactobacillus brevis, Lactobacillus fermentum, Lactobacillus fermentum RC-14, Lactobacillus fermentum B-54, Lactobacillus rhamnosus and Lactobacillus rhamnosus GR-1, octoxynol-9, chlorhexidine, benzalkonium chloride, nonoxynol-9, carrageenan, cyanovirin-N, fuzeon, hydroxyethyl cellulose, menfegol, dextran sulfate and cyclodextrin derivatives, and the like.

The amount of the therapeutically active agent incorporated in the delivery system varies depending on the particular therapeutically active agent, intended use of the substance, expected release rate and the time for which the system is expected to provide therapy. Since a variety of devices with varying sizes can be formulated for administering dosages, there is no critical upper limit on the amount of therapeutically active agent incorporated in the device. The lower limit depends on the activity of the therapeutically active agent and the expected release time. A person skilled in the art is readily able to determine the amount of the therapeutically active agent needed for each specific application of the delivery system.

Preferably, the amount of therapeutically active agent in the core varies between almost zero to 60 wt-%, when it is mixed into the polymer, the preferred amount being between 10-40 wt-%. Other possible ranges of the amount of the therapeutically active agent are 0.5-60 wt-%, 5-55 wt-%, 10-50 wt-%, 25-60 wt-%, 40-50 wt-% and 15-35 wt-%. The amount of the therapeutically active or the health promoting agent in the membrane varies between almost zero to 20 wt-%, the preferred amount being between 1-20 wt-%. Other possible ranges of the amount of the therapeutically active agent are 0.5-15 wt-%, 1-10 wt-%, 5-20 wt-%, 8-15 wt-%.

The daily dosage of the therapeutically active substances for a defined condition to be treated and for a defined substance can be achieved with the delivery system according to the invention particularly by varying the polymer composition of the matrix or membrane or both, for example so that the polysiloxane elastomer will contain a proper amount of poly(alkylene oxide) groups. An increasing concentration of such groups in the elastomer will increase the drug permeation. In addition to modifying the elastomer, other parameters such as the size and form of the device, the drug load, etc. will influence the daily dose released from said device. Some, but not undue, experimentation will be needed to find the most suitable parameters for each combination.

The daily dosage, i.e. the daily release rate needed for contraception is in the range of 1-5 mg for drospirenone, 0.005-0.050 mg for ethinyl estradiol, 0.050-0.200 mg for estradiol and, for example, 0.040-0.080 mg for gestodene. A preferred daily release rate for drospirenone is 2.5-3.5 mg, and more preferred release rate is 3 mg. For hormone therapy the corresponding values are in the range of 0.1-10 mg for drospirenone, 0.001-0.100 mg for ethinyl estradiol, 0.010-0.500 mg for estradiol and, for example, 0.005-0.200 mg for gestodene.

For lactobacillus species no official requirement concerning the therapeutic cell counts for the urogenital application exists. In the vaginal applications the cell counts may vary from 10⁶-10⁹ cfu/product.

Depending on the use of the device, the expected release time of progestogens and estrogens varies from one week to several months, for example from one week to 12 months, preferably from one week to 6 months and more preferably from 21 days to 3 months. The release time of additional therapeutically active agents or health-promoting agents may be shorter and may vary from one day up to 3 months, preferably from one day to 1 month and more preferably from one day to three weeks.

### Manufacture of the device

The drug delivery system according to this invention can be manufactured by any known techniques. The therapeutically active agent may be mixed within the core or membrane material, processed to the desired shape by moulding, injection moulding, casting, extrusion, such as co-extrusion and/or blend-extrusion or other appropriate methods. The membrane layer can be applied onto the core according to known methods, such as by mechanical stretching or expanding a prefabricated, tube formed membrane by pressurised gas, e.g. by air, swelling in a suitable solvent, for example such as cyclohexane, diglyme, propanol, isopropanol or a mixture of solvents, or by extrusion, moulding, spraying or dipping. The surface of a membrane or one of the membranes can be encased, coated or dusted by particles, crystals, microcrystals, powder or suspension of a therapeutically active or a health-promoting substance by using known methods, for example by spraying the whole delivery system or a part of it, i.e. a core or a compartment, with a suspension of said substance in a suitable solvent or by dipping the system in such a suspension.

An especially suitable method for preparation is disclosed in the Finnish patent FI 97947. This patent discloses an extrusion technology where prefabricated rods containing the active ingredient are coated by an outer membrane. A therapeutically active agent is mixed within the core matrix polymer composition, and processed to the desired shape and size by using known extrusion methods. The membrane layer may then be applied onto the prefabricated cores by feeding the cores to the extruder followed either by another core or a core without any active ingredient, i.e. by a placebo compartment, or by an empty space filled with air, which during the extrusion process will be filled with the membrane material to form a separation membrane. The drug-loaded core and the membrane layer can also be prepared simultaneously by co-extrusion.

The fibers or strings thus obtained can be cut into pieces of the required length and each piece can be assembled in any suitable manner to form a device shaped, sized and adapted for placing in the vagina. The device can have many shapes, for example various continuous, curved shapes, such as annular, ring-shaped, oval, spiral, ellipse, toroidal and the like. The cross section of the device can have almost any shape, and it can be for example circular, oval, flat, ellipse, star-shaped and the like.

The ends of the fiber or segments can be joined together to form a drug delivery device using a coupling means, which can be any method, mechanism, device or material known in the art for bonding or joining materials or structures together. The coupling can for example include solvent bonding, adhesive joining, heat fusing, heat bonding, pressure, and the like. When a solvent is used, the ends of the segments are moistened with an organic solvent that causes the surfaces to feel tacky, and when placed in contact the surfaces then bond and adhere in a fluid tight union. The ends of the fiber can be joined together by applying an adhesive or a sealant to at least one end of a segment, and then contacting the ends, or by placing the fiber in a mould at an elevated temperature (e.g. a temperature of above about 40°C), injecting molten high density polyethylene in between the fiber ends and cooling the prepared ring, or by joining the fiber ends together by welding.

Tubular compartments can also be joined into a closed system by using a plug or a stopper made of any inert, biocompatible material which does not permit the transport of active material. Examples of suitable impermeable material are metals, such as gold, silver or silver alloys, glass or ceramic material and suitable polymers. If desired, a biocompatible adhesive can be used for better sealing or better adhesion of the plug or stopper to the compartment.

The delivery system can also comprise a substantially inert supporting means made of a material which is biologically compatible and remains unchanged for a sufficient period of time in the conditions prevailing in the vagina. The term "substantially inert" means in this connection that the active agent cannot, to any substantial degree, diffuse or in any other way migrate from the core into the support means. Suitable supporting materials are for example cross-linked rubbers, such as e.g. natural rubber, butyl rubber and polydimethylsiloxane elastomers, flexible thermoplastic resins, such as ethyl vinyl acetate (EVA), thermoplastic polymers, such as styrene copolymers, polyurethanes, thermoplastic polyolefins and inert, biocompatible metals.

The support means can be prepared in a simple, known manner. A suitable polymeric material may for example be compressed in a mould, or extruded to form a rod-like member with a suitable diameter, then followed by cutting the extrudate to pieces of suitable length and by vulcanizing into the desired, substantially annular shape. The supporting member can be of solid material or hollow.

One or more ring sections, membranes or cores may be assembled on the prefabricated closed, continuous supporting member in the form of layers or coatings. Drug containing cores can for example be prepared by incorporating the finely ground or even micronized active substance in the polymer composition to form a suspension, which is then applied as a layer on the supporting means by using known techniques, such as spraying, dipping or the multi-colour injection moulding technique, and vulcanized by known methods. Membrane or membranes can be assembled in a similar way.

Alternatively the hollow, sleeve-like core or cores are mounted on the rod-like supporting means preferably by first enlarging the diameter to some degree and thereafter by simply sliding them onto the supporting means or inserting the supporting means into the hollow cores. When the cores are of a silicone based polymer or a cross-linked rubber the enlargement can take place, for example, by swelling in a suitable organic solvent, whereafter the swollen body is mounted onto the supporting means. When the solvent evaporates, the cores tighten onto the support means. As an alternative, the tube-like core can be stretched mechanically with a suitable device or by using for example pressurized gas and threaded in the stretched state onto the support means. When the stretching force is discontinued, the sleeve-like body is tightened onto the support means. Membrane or membranes can be assembled by mounting a suitable polymer tube on an individual core or on a rod-like delivery system using for example solvent swelling or mechanical stretching. Finally the ends of the rod or the string so obtained are joined by using known techniques.

The delivery system according to the invention can be manufactured in any size as required, the exact size is being dependent on the mammal and particular application. In practice, for a human female an outer ring diameter is typically from 35 to 70 mm, preferably from 35 to 58 mm or from 45 to 65 mm and more preferably from 50 to 58 mm. The cross sectional diameter is typically from 1 to 10 mm. In a particular embodiment the cross sectional diameter is between 2 and 6 mm, in a specific embodiment between about 3.0 and 5.5 mm and in another embodiment between about 3.5 and 4.5 mm and in yet another embodiment is 4.0 or 5.0 mm.

The lengths of the cores of the drug delivery system are chosen to give the required performance. Ratios of the lengths of the cores will depend upon the particular therapeutic application, including the desired ratio and dosages of each drug to be delivered. The length of the drug containing compartments can be for example from 5 to 160 mm, or up to the total length of the delivery system. The length of each placebo compartment separating the drug containing cores may generally vary between 2-110 mm and depends on the nature of the material and its capacity to prevent permeation of the active materials. Most ideally the placebo compartment completely prevents mixing of the active substances, which otherwise might disturb the release pattern.

The thickness of a separation membrane can be about 0.2 to 5 mm. The layer containing the active substance may have a thickness of 0.1 to 5.0 mm, and preferably 0.2 to 3.5 mm. The thickness of the membrane is from 0.1 to 1.0 mm, preferably 0.2 to 0.6 mm.

### EXPERIMENTAL PART

### Drug release test

The release rate of the drug from the device is measured in vitro as follows: The delivery systems are attached into a stainless steel holder in vertical position and the holders with the devices are placed into glass bottles containing 250 ml or less of a medium. The glass bottles are shaken in shaking water bath 100 rpm at 37 °C. The dissolution medium is withdrawn and replaced by a fresh dissolution medium at predetermined time intervals, and the amount of the released drug is analysed by using standard HPLC methods. The concentration of the dissolution medium and the moment of change (withdrawal and replacement) of medium are selected so that sink-conditions are maintained during the test. Frequency of sampling is chosen to keep sink conditions in the medium.

The invention as well as measured release rates, which were at the expected level, are further illustrated by the following, non-limiting examples.

The delivery systems of the examples are manufactured in accordance with standard techniques known in the art and described in the patent application. The therapeutically active agent is mixed within the core matrix polymer composition, and processed to the desired shape by using known methods. The membrane is made and assembled onto the cores according to known methods, such as by expanding the prefabricated, tube formed membrane in a suitable solvent, for example such as propanol, isopropanol, or by using a coextrusion method described in the Finnish patent FI 97947. According to said method, each of the cores are fed to the extruder followed either by an empty space filled with air or by another core without any active ingredient. The ends of the fabricated rods comprising the cores and the membrane are joined together by using a plug or a sealant. Silica is preferably used as a filler.

### Example 1

### A delivery system for simultaneous administration of drospirenone, estradiol and lactic acid.

A device comprising drospirenone at a target release rate of 1.6 mg/day and estradiol at a target release rate of 120 gg/day is prepared. The core containing drospirenone consists of the composition containing PEO-b-PDMS copolymer (25 wt-% of the total amount of polymers) and PDMS and the length of the core is 100 mm. The second core comprising estradiol consists of PEO-b-PDMS copolymer (24 wt-% of the total amount of polymers) and PDMS and the length is 15 mm. The outer diameter of the cores is 3.0 mm. Two placebo compartments added to separate the drug containing cores consist of PDMS and their length is 20 and 35 mm. The content of drospirenone and estradiol in the core are 40 wt-% and 18 wt-%, respectively. The membrane comprising 5 wt-% of lactic acid is made of PEO-b-PDMS copolymer (15 wt-%) and PDMS (85 wt-%). The wall of the membrane tube is 0.25 mm, inner diameter 2.85-2.9 mm and the outer diameter 3.35-3.4 mm. The cores and the tube-formed membrane are made by extrusion. The core is coated by the membrane by first swelling the membrane in propanol. The ends of the delivery system are joined into a ring by using a polyethylene plug.

### Example 2

### A delivery system for simultaneous administration of drospirenone, estradiol hemihydrate and polylactic acid

A device comprising drospirenone at a target release rate of 5.0 mg/day and estradiol hemihydrate at a target release rate of 150 gg/day is prepared. The first core comprising drospirenone (38 wt-%) consists of PEO-b-PDMS (41 wt-% of the total amount of polymers) and PDMS and the length of the core is 130 mm. The second core comprising estradiol (20 wt-%) consists of PEO-b-PDMS (40 wt-% of the total amount of polymers) and PDMS, and the length is 10 mm. The outer diameter of the core is 3.6 mm. An inert core consisting of PDMS is added to give a rod having the total length of 180 mm. The core parts are encased in a membrane consisting of PEO-b-PDMS/PDMS in a ratio of 20:80 and containing 10 wt-% of polylactic acid. The membrane layer is applied onto the prefabricated cores by using coextrusion. An empty space of 3 mm left between the drug containing cores is during the process filled by the membrane material thus forming a separation membrane. The thickness of the membrane wall is 0.3 mm, the inner diameter of the tube 3.4 mm and the outer diameter 4.0-4.05 mm.

### Example 3

### A delivery system for simultaneous administration of gestodene, estradiol and Carbopol

A device comprising gestodene at a target release rate of 60 µg/day and estradiol at a target release rate of 20 µg/day is prepared. The first part of the core consists of PDMS comprising gestodene and the length is 45 mm. The second part of the core consisting of PEO-b-PDMS (20 wt-% of the total amount of polymers) and PDMS, comprises estradiol and the length is 30 mm. The outer diameter of the cores is 2.58 mm. Two 50 mm placebo compartments consisting of PDMS are used to separate the drug containing cores. The core parts are encased in a membrane consisting of PDMS and methyltrifluoropropyl-methylvinyl siloxane in a ratio of 70:30. The thickness of the membrane wall is 0.25 mm, inner diameter 2.40 mm and the outer diameter 2.9 mm. The ends of the delivery system are joined to a closed system, and finally the system is dipped in Carbopol suspension to give a thin polymer coating.

### Example 4

### A delivery system for simultaneous administration of levonorgestrel, ethinyl estradiol and Lactobacillus

A delivery system comprising levonorgestrel at a target release rate of 50 µg/day and ethinyl estradiol at a target release rate of 20 µg/day is prepared. The first core comprising levonorgestrel consists of PDMS and the length of the core is 40 mm. The second core comprising estradiol consists of PEO-b-PDMS (18 wt-% of the total polymer amount) and PDMS, and the length of the core is 15 mm. The cores are separated by inert placebo cores consisting of a siloxane based elastomer comprising 3,3,3-trifluoropropyl groups attached to the siloxane silicon atoms (degree of trifluoropropyl substitution is 49.5 %), the length of the cores are 10 mm and 100 mm. The outer diameter of the cores is 2.6-2.7 mm. The cores are encased in a membrane consisting of PEO-b-PDMS/PDMS in a ratio of 10:90. The thickness of the membrane wall is 0.22 mm, inner diameter of the tube is 2.35 mm and the outer diameter approximately 2.8 mm. The ends of the delivery system are sealed with silicon glue. The outer surface of the membrane is coated with a thin layer of Lactobacillus rhamnosus.

### Example 5

### A delivery system for simultaneous administration of drospirenone, estradiol and carbopol

A device comprising drospirenone at a target release rate of 3.0 mg/day and estradiol at a target release rate of 100 µg/day is prepared. The first core comprising drospirenone (35 wt-%) consists of PEO-b-PDMS (41 wt-% of the total polymer amount) and PDMS, and the length of the core is 130 mm. The second core comprising estradiol (18 wt-%) consists of PEO-b-PDMS (25 wt-% of the total polymer amount) and PDMS, and the length of the core is 10 mm. The outer diameter of the core is 3.6 mm. An inert core consisting of PDMS is added to give a rod having the total length of 180 mm. The core parts are encased in a membrane consisting of PEO-b-PDMS/PDMS in a ratio of 10:90 and containing 5 wt-% of carbopol. The membrane layer is applied onto the prefabricated cores by using coextrusion. An empty space of 3 mm left between the drug containing cores is during the process filled by the membrane material thus forming a separation membrane. The thickness of the membrane wall is 0.35 mm, the inner diameter of the tube is 3.45 mm and the outer diameter is 4.15-4.2 mm.

### Example 6

### A delivery system for simultaneous administration of drospirenone, ethinyl estradiol and polyglycolic acid

A device comprising drospirenone at a target release rate of 2.5 mg/day and ethinyl estradiol at a target release rate of 15 µg/day is prepared. The first core comprising drospirenone (30 wt-%) consists of PEO-b-PDMS (35 wt-% of the total polymer amount) and PDMS and the length of the core is 120 mm. The second core comprising ethinyl estradiol (10 wt-%) consists of PDMS, and the length is 10 mm. The outer diameter of the core is 3.5 mm. Inert cores of 20 mm and 25 mm consisting of PDMS are added to separate the drug containing cores. The core parts are encased in a membrane consisting of PEO-b-PDMS/PDMS in a ratio of 33:67. The thickness of the membrane wall is 0.3 mm, the inner diameter of the tube is 3.35-3.4 mm and the outer diameter is 3.95-4.0 mm. The ends of the delivery system are joined into a ring by using a polyethylene plug, and finally the delivery system is dipped in polyglycolic acid suspension to give a thin coating.

### Example 7

### A delivery system for simultaneous administration of drospirenone, estradiol valerate and Lactobacillus

A device comprising drospirenone at a target release rate of 3 mg/day and estradiol valerate at a target release rate of 150 µg/day is prepared. The first core comprising drospirenone (30 wt-%) consists of PEO-b-PDMS (45 wt-% of the total polymer amount) and PDMS, and the length of the core is 130 mm. The second core comprising estradiol valerate (10 wt-%) consist of PEO-b-PDMS (15 wt-% of the total polymer amount) and PDMS, and the length of the core is 20 mm. The outer diameter of the core is 3.5 mm. Inert cores of 8 mm and 10 mm consisting of PDMS are added to separate the drug containing cores. The core parts are encased in a membrane consisting of PEO-b-PDMS/PDMS in a ratio of 20:80. The thickness of the membrane wall is 0.3 mm, the inner diameter of the tube is 3.3-3.35 mm and the outer diameter is 3.9-3.95 mm. The ends of the delivery system are joined together into a closed system by using a glass plug, and finally the system is dipped in the suspension of Lactobacillus reuterii RC-14 to give a thin coating.

Although the invention has been described in terms of particular embodiments and applications, one of ordinary skill in the art can in the light of this teaching generate additional embodiments and modifications without departing from the spirit of or exceeding the scope of the claimed invention. Accordingly, it is to be understood that the descriptions herein are offered by way of example to facilitate comprehension of the invention and should not be construed to limit the scope thereof.

## Claims

1. An intravaginal delivery system for the controlled release of a progestogen and an estrogen over a prolonged period of time, the system consisting of one or more compartments, which one or each comprise a core and a membrane encasing the core, said core and membrane consisting essentially of a same or different polymer composition, **characterized in that** the delivery system additionally comprises a substance capable of giving and/or enhancing the protection against bacterial and fungal infections and/or enhancing the protection against sexually transmitted diseases.

2. An intravaginal delivery system according to claim 1 consisting of one compartment comprising a mixture of a progestogen and an estrogen, **characterized in that** the membrane comprises a substance capable of giving and/or enhancing the protection against bacterial and fungal infections, and/or enhancing the protection against sexually transmitted diseases.

3. An intravaginal delivery system according to claim 1 consisting of one compartment comprising a mixture of a progestogen and an estrogen, **characterized in that** the core additionally comprises a substance capable of giving and/or enhancing the protection against bacterial and fungal infections, and/or enhancing the protection against sexually transmitted diseases.

4. An intravaginal delivery system according to claim 1 consisting of two or more compartments, wherein at least one of the compartments comprises a progestogen and another compartment comprises an estrogen or a mixture of a progestogen and an estrogen, **characterized in that** the membrane of at least one of the cores comprises a substance capable of giving and/or enhancing the protection against bacterial and fungal infections, and/or enhancing the protection against sexually transmitted diseases.

5. An intravaginal delivery system according to claim 1 consisting of two or more compartments, wherein at least one of the compartments comprises a progestogen and another compartment comprises an estrogen or a mixture of a progestogen and an estrogen, **characterized in that** at least one of the compartments comprises a substance capable of giving and/or enhancing the protection against bacterial and fungal infections, and/or enhancing the protection against sexually transmitted diseases.

6. An intravaginal delivery system according to any of the claims 1-5, **characterized in that** the progestogen is selected from the group of cyproterone acetate, desogestrel, etonogestrel, levonorgestrel, lynestrenol, medroxyprogesterone acetate, norethisterone, norethisterone acetate, norgestimate, gestodene, drospirenone or any other compound having a progestogenic activity.

7. An intravaginal delivery system according to claim 6, **characterized in that** the progestogen or a drug having a progestogenic activity is drospirenone.

8. An intravaginal delivery system according to any of the claims 1-7, **characterized in that** the estrogen is selected from the group consisting of estradiol, ethinyl estradiol, estradiol esters, estradiol hemihydrate, estradiol sulfamates, estriol succinate and conjugated estrogens, including conjugated equine estrogens such as estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequilenin sulfate and 17α-dihydroequilenin sulfate or mixtures thereof.

9. An intravaginal delivery system according to claim 8, **characterized in that** the estrogen is estradiol, estradiol hemihydrate, estradiol valerate, estradiol succinate, estradiol benzoate, ethinyl estradiol, or estradiol sulfamate.

10. An intravaginal delivery system according to any of the claims 1-9, **characterized in that** the substance capable of giving and/or enhancing the protection against bacterial and fungal infections and/or enhancing the protection against sexually transmitted diseases is selected from the group of antimicrobial substances, antifungal substances, antibacterial substances, antiviral substances, vitamins, minerals, enzymes, co-enzymes, co-factors, microorganisms, organic acids and a variety of molecules extracted from natural sources such as amino acids, polysaccharides, peptides, naturally occurring hormones and biochemical intermediates

11. An intravaginal delivery system according to claim 10, **characterized in that** the substance capable of giving and/or enhancing the protection against bacterial and fungal infections, and/or enhancing the protection against sexually transmitted diseases is lactic acid, polylactic acid, glycolic acid, polyglycolic acid, carbopol, polycarbophil, ascorbic acid, D-pantothenic acid, folic acid and the reduced forms thereof, especially tetrahydrofolates and metabolites of folic acid, preferably 5-methyl-6(S)-tetrahydrofolic acid and its salts such as earth alkaline salts, especially the calcium salt (Metafolin), fumaric acid, benzoic acid, p-aminobenzoic acid, alginic acid, sorbic acid, tartaric acid, edetic acid or salts of the acids, niacinamide, Lactobacillus species, for example such as Lactobacillus reuteri, Lactobacillus reuterii RC-14, Lactobacillus delbrueckii, Lactobacillus gasseri, Lactobacillus jensenii, Lactobacillus catenaforme, Lactobacillus paracasei, Lactobacillus paracasei Lbp PB01, Lactobacillus casei, Lactobacillus acidophilus, Lactobacillus acidophilus Lba EB01, Lactobacillus crispatus, Lactobacillus crispatus CTV05, Lactobacillus salivarius, Lactobacillus brevis, Lactobacillus fermentum, Lactobacillus fermentum RC-14, Lactobacillus fermentum B-54, Lactobacillus rhamnosus and Lactobacillus rhamnosus GR-1, octoxynol-9, chlorhexidine, benzalkonium chloride or nonoxynol-9.

12. An intravaginal delivery system according to any of the claims 1-11, **characterized in that** said polymer compositions are selected from the group consisting of polydimethyl siloxanes, modified polydimethyl siloxanes, ethylene/vinyl acetate copolymers (EVA), polyethylene, polypropylene, acrylic acid polymers, polytetrafluoroethylene (PTFE), polyurethanes, poly(methacrylate), polymethyl methacrylate, poly(hydroxyethylmethacrylate) (pHEMA), polyhydroxy alkanoates, poly(lactic acid), poly(glycolic acid), hydrophilic polymers such as the hydrophilic hydrogels , cross-linked polyvinyl alcohol or a combination thereof.

13. An intravaginal delivery system according to any of the claims 1-11, **characterized in that** said elastomer compositions are selected from the group consisting of
- an elastomer composition comprising poly(dimethylsiloxane),
- an elastomer composition comprising a siloxane-based elastomer comprising 3,3,3-trifluoropropyl groups attached to the silicon atoms of the siloxane units,
- an elastomer composition comprising poly(alkylene oxide) groups, said poly(alkylene oxide) groups being present as alkoxy-terminated grafts or blocks linked to the polysiloxane units by silicon-carbon bonds, or a mixture of these forms, and
- a combination of at least two thereof.

14. An intravaginal delivery system according to claim 13, **characterized in that** in the polymer composition the amount of polydimethylsiloxane comprising poly(alkylene oxide) groups is from 5 to 80 wt-% of the total amount of polymers.

15. An intravaginal delivery system according to claims 13 and 14, **characterized in that** the poly(alkylene oxide) groups are poly(ethylene oxide) groups.

16. An intravaginal delivery system according to claim 13, **characterized in that** in the siloxane-based elastomer from 1 to approximately 50 % of the substituents attached to the silicon atoms of the siloxane units are 3,3,3-trifluoropropyl groups.

17. An intravaginal delivery system according to any of the claims 1-16, **characterized in that** in the system comprising two or more compartments, at least two of said compartments are adjacent to each other.

18. An intravaginal delivery system according to any of the claims 1-16, **characterized in that** in the system comprising two or more compartments, at least two of said compartments are separated by an inert space consisting essentially of a same or different polymer composition.

19. An intravaginal delivery system according to any of the claims 1-16, **characterized in that** in the system comprising two or more compartments, at least two of the said compartments are separated by a separation membrane consisting essentially of a same or different polymer composition.

20. An intravaginal delivery system according to any of the claims 1-19, **characterized in that** the membrane comprises at least two layers, each consisting of a same or different polymer composition.

21. An intravaginal delivery system according to any of the claims 1-20, **characterized in that** the surface of at least one membrane comprises a substance capable of giving and/or enhancing the protection against bacterial and fungal infections, and/or enhancing the protection against sexually transmitted diseases.

22. A method of giving and/or enhancing the protection for the female mammal against vaginal bacterial and fungal infection and/or enhancing the protection against sexually transmitted diseases by using an intravaginal delivery system intended for the controlled release of progestogen and an estrogen at a level required for contraception or hormone replacement therapy, said method comprising the steps of positioning the delivery system within the female vaginal tract and retaining the system a prolonged period of time within the vaginal tract, preferably for at least approximately 21 days, **characterized in that** said delivery system additionally releases a sufficient amount of a substance capable of giving and/or enhancing the protection against bacterial and fungal infections and/or enhancing the protection against sexually transmitted diseases.
